(19) Europäisches Patentamt
European Patent Office
Office européen des brevets

(11) **EP 3 913 424 A1**

(12) **EUROPÄISCHE PATENTANMELDUNG**

(43) Veröffentlichungstag:
**24.11.2021 Patentblatt 2021/47**

(51) Int Cl.:
***G02C 13/00*** *(2006.01)* ***A61B 3/11*** *(2006.01)*

(21) Anmeldenummer: **20176093.1**

(22) Anmeldetag: **22.05.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Carl Zeiss Vision International GmbH 73430 Aalen (DE)**

(72) Erfinder:
• **GROMOTKA, Jeremias 73433 Aalen (DE)**
• **GAMPERLING, Michael 89340 Leipheim (DE)**

(74) Vertreter: **Sticht, Andreas Kraus & Weisert Patentanwälte PartGmbB Thomas-Wimmer-Ring 15 80539 München (DE)**

(54) **COMPUTERIMPLEMENTIERTES VERFAHREN ZUR BESTIMMUNG VON ZENTRIERPARAMETERN FÜR MOBILE ENDGERÄTE, MOBILES ENDGERÄT UND COMPUTERPROGRAMM**

(57) Es werden Verfahren und Vorrichtungen zum Bestimmen mindestens eines Zentrierparameters bereitgestellt. Dabei wird ein mobiles Endgerät von einer ersten Position zu einer zweiten Position bewegt, und es wird jeweils ein Bild von einer Augenpartie einer Person aufgenommen. Zudem wird bei der Bewegung die Beschleunigung gemessen. Der Zentrierparameter wird dann auf Basis der aufgenommenen Bilder sowie der gemessenen Beschleunigung bestimmt.

Fig. 4

EP 3 913 424 A1

**Beschreibung**

Die vorliegende Anmeldung betrifft computerimplementierte Verfahren zur Bestimmung von Zentrierparametern für ein mobiles Endgerät, entsprechende mobile Endgeräte sowie Computerprogramme.

Unter einem mobilen Endgerät ist eine Vorrichtung zu verstehen, welche zumindest einen programmierbaren Prozessor, eine Anzeige sowie eine Kamera und einen Beschleunigungssensor umfasst, und welche dazu ausgelegt ist, getragen zu werden, das heißt hinsichtlich Dimensionen und Gewicht derart ausgestaltet ist, dass es von einer Person mitführbar ist. Typische Beispiele für derartige mobile Endgeräte sind Smartphones oder Tablet-PCs, die heutzutage bei fast allen erhältlichen Modellen über einen Sensorbildschirm (üblicherweise als Touchscreen bezeichnet), eine oder mehrere Kameras, Beschleunigungssensoren und andere Sensoren wie Gyroskopsensoren und weitere Komponenten wie drahtlose Schnittstellen für Mobilfunk oder WLAN (Wireless LAN) aufweisen. Das Gewicht derartiger mobiler Endgeräte liegt typischerweise zumindest unter 2 kg, meist unter 1 kg und noch weit darunter. Computerprogramme für derartige mobile Endgeräte werden üblicherweise als Apps (kurz für das englische Wort "Application", das heißt Anwenderprogramm) bezeichnet.

Um Brillengläser korrekt in einer Brillenfassung anzuordnen, das heißt zu zentrieren, sodass die Brillengläser in korrekter Position relativ zu den Augen einer Person, welche die Brille benutzen soll, getragen werden, werden sogenannte Zentrierparameter benutzt. Einige dieser Zentrierparameter sind beispielsweise in Kapitel 5 der Norm DIN EN ISO 13666: 2012 definiert und umfassen beispielsweise den Pupillenabstand PD gemäß Punkt 5.29 dieser Norm. Ein anderer dieser Zentrierparameter ist die Kastenhöhe des in 5.1 dieser Norm erwähnten Kastensystems, welches in der Norm DIN EN ISO 8624:2015 genauer definiert ist.

In den letzten Jahren wurden Vorrichtungen und Verfahren entwickelt, mit welchen derartige Zentrierparameter automatisch bestimmbar sind. So ist das Zeiss Visufit 1000 ein Zentriersystem, welches ein in sich kalibriertes Kamerasystem benutzt, um zumindest eine Augenpartie eines Kopfes einer Person mit getragener Brillenfassung aus mehreren Richtungen aufzunehmen und hieraus die Zentrierparameter zu bestimmen. Dieses Gerät ist stationär und zur Anwendung bei einem Optiker gedacht.

Andere Herangehensweisen benutzen einen Messbügel, der an der Brillenfassung angebracht wird und als Referenz dient. Derartige Herangehensweisen sind aus der US 9 928 421 B1, der US 9 971 172 B1, der US 7 950 800 B1, der US 8 360 580 B1, der US 7 950 800 B1 oder der US 9 535 270 B2 bekannt.

In der US 9 928 421 B1 wird dabei eine Person, die eine Brille und einen Messbügel trägt, aus verschiedenen Richtungen aufgenommen. Bei den Aufnahmen wird ein Neigungswinkel der verwendeten Kamera für die jeweiligen Aufnahmen ermittelt, damit eine geneigte Anordnung der Kamera berücksichtigt werden kann. Aus den Aufnahmen werden dann Zentrierparameter bestimmt.

Die US 9 9771 172 B1 offenbart ein Zentriergerät, bei dem auf eine Bildebene projizierte Dimensionen einer Brillenfassung in Bezug auf eine Kopfhaltung korrigiert werden können, um auch bei größeren Neigungswinkeln einer verwendeten Kamera und Drehwinkeln des Kopfes möglichst genau Zentrierdaten bestimmen zu können. Dabei wird ein einziges Frontbild des Kopfes verwendet, das heißt eine Bildaufnahme von vorne, wobei benötigte Neigungs- und Drehwinkel aus der Abbildung eines Messbügels bestimmt werden.

Die US 7 950 800 B1 offenbart eine Bestimmung von Zentrierdaten, bei der wiederum ein Messbügel verwendet wird, um einen Maßstab für Aufnahmen durch eine Kamera zu bieten, die eine Frontansicht einer Person aufnimmt. Dabei wird wiederum eine Neigung der Kamera berücksichtigt.

Die US 8 360 580 B1 beschreibt, wie mit Hilfe einer Bildaufnahme durch eine Kamera und der Verwendung von Markierungselementen am Kopf einer Person der sogenannte Augendrehpunkt bestimmt werden kann. Die Lage des Augendrehpunktes ist bei der Berechnung von Zentrierparametern dann relevant, wenn die zum Zeitpunkt der Aufnahme eingenommene Blickrichtung nicht mit der sogenannten Fernblickrichtung übereinstimmt, bei der die Person ihren Blick im Wesentlichen in das Unendliche richtet. In dieser Druckschrift wird die Lage des Augendrehpunkts mit Hilfe von zwei Aufnahmen bestimmt, die die Person bei unterschiedlichen Blickrichtungen abbilden. Markierungen am Kopf werden benutzt, um absolute Größen bestimmen zu können.

Auch die US 7 950 800 B1 beschreibt Verfahren zur Bestimmung von Zentrierparametern mit einer Hand gehaltenen Kamera, bei der wiederum ein Messbügel als absoluter Größenmaßstab verwendet wird.

Die US 9 535 270 B2 offenbart ein Verfahren, bei dem eine feststehende Videokamera eine Person aufnimmt, während die Person den Kopf hebt und dabei ein feststehendes Ziel beachtet. Ein Signal eines Neigungsmessers wählt dabei aus der so entstehenden Serie von Bildaufnahmen während des Hebens des Kopfes diejenige aus, die eine normale Kopfhaltung am besten wiedergibt. Dabei wird das Gesicht von einer Frontkamera eines Tablet-PCs aufgenommen. Ein Messbügel, den die Person trägt, wird von beiden Kameras, das heißt der Videokamera und der Frontkamera des Tablet-PCs, abgebildet. Die beiden Kameras bilden dabei einen Winkel von ca. 30°. Aus den Bildaufnahmen werden dann Zentrierparameter berechnet.

Die oben beschriebenen Verfahren und Vorrichtungen benötigen zwingend einen Messbügel (oder anderen Maßstab) oder ein Mehrkamerasystem und sind grundsätzlich zur Durchführung durch einen Optiker oder eine andere entsprechend geschulte Person bestimmt. Beide Verfahren sind in erster Linie für den stationären Bereich und nur bedingt für mobile Endgeräte geeignet.

Es besteht ein Bedarf nach vereinfachten Herangehensweisen zur Bestimmung zumindest mancher Zentrierparameter, welche mittels eines mobilen Endgeräts wie eines Smartphones oder Tablet-PCs durchgeführt werden können und welche insbesondere von einer zu untersuchenden Person selbst ohne größeren Aufwand durchgeführt werden können. Ausgehend von Verfahren und Vorrichtungen, welche Messbügel verwenden, beispielsweise wie in der US 7 950 800 B1, ist es dabei eine Aufgabe, Verfahren und Vorrichtungen bereitzustellen, bei denen derartige Messbügel oder andere Hilfsmittel zumindest nicht zwingend erforderlich sind.

Diese Aufgabe wird gelöst durch ein computerimplementiertes Verfahren nach Anspruch 1, ein entsprechendes mobiles Endgerät nach Anspruch 15 sowie ein Computerprogramm nach Anspruch 14. Die abhängigen Ansprüche definieren weitere Ausführungsbeispiele sowie ein Verfahren zum Einschleifen von Brillengläsern.

Erfindungsgemäß wird ein computerimplementiertes Verfahren für ein mobiles Endgerät bereitgestellt, umfassend:

Aufnehmen eines ersten Bildes zumindest einer Augenpartie einer Person mit einer Kamera des mobilen Endgeräts an einer ersten Position des mobilen Endgeräts,
Aufnehmen eines zweiten Bildes der Augenpartie der Person mit der Kamera an einer zweiten Position des mobilen Endgeräts, und
Bestimmen von mindestens einem Zentrierparameter auf Basis des ersten Bildes und des zweiten Bildes.

Das Verfahren ist gekennzeichnet durch wiederholtes Messen einer Beschleunigung des mobilen Endgeräts bei einer Bewegung des mobilen Endgeräts von der ersten Position zu der zweiten Position. Der mindestens eine Zentrierparameter wird dann zudem auf Basis der wiederholt gemessenen Beschleunigung ermittelt.

Das zweite Bild muss nicht unmittelbar nach dem ersten Bild aufgenommen werden. Beispielsweise kann eine Videosequenz mit einer Vielzahl von Bildern, darunter das erste Bild und das zweite Bild, aufgenommen werden, wobei zwischen dem ersten Bild und dem zweiten Bild ein weiteres Bild oder mehrere weitere Bilder der Vielzahl von Bildern liegen können. Zudem oder alternativ können auch vor dem ersten Bild oder nach dem zweiten Bild ein weiteres Bild oder mehrere weitere Bilder der Vielzahl von Bildern liegen. Das Verfahren kann auch zusätzlich derartige weitere Bilder und zugehörige Positionen und Beschleunigungen zur Bestimmung von Zentrierparametern heranziehen.

Der mindestens eine Zentrierparameter kann den Pupillenabstand umfassen. Trägt die Person eine Brillenfassung, kann der mindestens eine Zentrierparameter zudem oder alternativ eine Höhe des Kastensystems der Brillenfassung oder andere Maße des Kastensystems der Brillenfassung umfassen. So kann der Pupillenabstand oder Maße des Kastensystems einfach bestimmt werden.

Die Augenpartie einer Person ist ein Teil des Gesichts der Person, die zumindest die Augen der Person sowie, falls die Person eine Brillenfassung trägt, eine von der Person getragene Brillenfassung beinhaltet. Unter einer Brillenfassung ist dabei in Übereinstimmung mit DIN ESO 77998:2006-01 und DIN ESO 8624:2015-12 ein Gestell oder eine Halterung zu verstehen, mittels dem/der Brillengläser am Kopf getragen werden können. Der Begriff wie hier verwendet beinhaltet insbesondere auch randlose Brillenfassungen. Brillenfassungen werden umgangssprachlich auch als Brillengestelle bezeichnet.

Bei einem derartigen Verfahren ist ein zusätzlicher Maßstab wie ein Messbügel oder Messpunkte an einem Kopf der Person nicht nötig. Durch das wiederholte Messen der Beschleunigung kann durch zweifaches Integrieren der Beschleunigung ein Weg des mobilen Endgeräts von der ersten Position zu der zweiten Position bestimmt werden, insbesondere ein Abstand zwischen der ersten Position und der zweiten Position. Dies ermöglicht dann die Bestimmung des mindestens einen Zentrierparameters ohne Verwendung eines Maßstabs. Unter einem wiederholten Messen wird dabei ein Messen mit einer Rate, d.h. einer Anzahl von Messwerten pro Zeiteinheit, verstanden, so dass der Weg des mobilen Endgeräts hinreichend genau bestimmt werden kann, um die Zentrierparameter genau genug für die Brillenglaszentrierung bestimmen zu können. Bei üblichen mobilen Endgeräten erfolgt eine digitale Verarbeitung der mit einem eingebauten Beschleunigungssensor des mobilen Endgeräts gemessenen Beschleunigung mit einer Abtastrate. Für das erfindungsgemäße Verfahren kann eine Abtastrate von 10Hz, d.h. 10 erfasste Messwerte pro Sekunde, oder sogar darunter ausreichend sein. Dabei können die Messwerte tiefpassgefiltert werden, um die Genauigkeit zu erhöhen. Höhere Abtastraten sind ebenso möglich und können die Genauigkeit erhöhen. Nach oben hin ist die Abtastrate durch die Geschwindigkeit der Hardwarekomponenten des mobilen Endgeräts begrenzt. Durch hohe Abtastraten kann dabei eine kontinuierliche Messung angenähert werden.

Eine Kamera und einen Sensor zum Messen der Beschleunigung ist praktisch in jedem kommerziell erhältlichen mobilen Endgerät wie einem Smartphone oder einem Tablet-PC vorhanden. Somit kann durch einfaches Bereitstellen eines Computerprogramms für das mobile Endgerät, das heißt einer App, das obige Verfahren implementiert werden, ohne dass zusätzliche Hardware benötigt wird.

Zum Erfassen des mindestens einen Zentrierparameters kann die Lage der Pupillen in dem ersten Bild und dem zweiten Bild und/oder die Brillenfassung in dem ersten Bild und zweiten Bild mit herkömmlichen Verfahren der Bildverarbeitung identifiziert werden. Für den Fassungsrand einer Brillenfassung sind solche Verfahren beispielsweise in der EP 3 355 214 A1 beschrieben. Zur Detektion der Lage der Pupillen im Bild sind entsprechende Verfahren in S. Kim et al., "A Fast Center of Pupil Detection Algorithm for VOG-Based Eye Movement Tracking",Conf Proc IEEE Eng Med Biol Soc. 2005;3:3188-91 oder in Mansour Asadifard und Jamshid Shanbezadeh, "Automatic Adaptive Center of Pupil Detection Using Face Detection and CDF Analysis" , Proceedings of the International MultiConference of Engineers and Computer Scientists 2010 Vol I, IMECS 2010, 17.-19. März 2010, Hongkong, beschrieben.

Bevorzugt erfolgt die Bestimmung des mindestens einen Zentrierparameters zudem auf Basis von Bildwinkeleigenschaften der Kamera. Diese Bildwinkeleigenschaften geben an, auf welchen Bildpunkten eines Bildsensors der Kamera ein Objekt abgebildet wird, das unter einem bestimmten Winkel zur optischen Achse der Kamera steht. In anderen Worten geben die Bildwinkeleigenschaften eine Korrelation zwischen diesem Winkel zur optischen Achse und dem Bildpunkt an. Die optische Achse bezeichnet dabei bei der Kamera eine Symmetrieachse des optischen Systems der Kamera, welches üblicherweise rotationssymmetrisch ist.

Derartige Bildwinkeleigenschaften ermöglichen also eine Zuordnung zwischen Bildpunkten, in denen Objekte wie beispielsweise die Pupillen aufgenommen werden, und Winkeln, was eine Berechnung entsprechender Zentrierparameter auf einfache Weise ermöglicht.

Die Bildwinkeleigenschaften der Kamera können beispielsweise aus Herstellerangaben bezüglich des Bildwinkels eines Objektivs der Kamera bestimmt werden. Sie können aber auch im Rahmen des computerimplementierten Verfahrens vorab bestimmt werden, indem ein erstes Bestimmungsbild und ein zweites Bestimmungsbild aufgenommen mit der Kamera aufgenommen werden, wobei das mobile Endgerät zwischen der Aufnahme des ersten Bestimmungsbildes und des zweiten Bestimmungsbildes verdreht wird. In dem ersten und zweiten Bestimmungsbild werden dann mittels Bildverarbeitung einander entsprechende Objekte (das heißt Objekte, die in beiden Bildern sichtbar sind) identifiziert. Aus der Verdrehung, die mit einem Orientierungs- oder Beschleunigungssensor des mobilen Endgeräts gemessen werden kann, und daraus, auf welchen Bildpunkten die Objekte im ersten und zweiten Bestimmungsbild abgebildet werden, können dann die Bildwinkeleigenschaften bestimmt werden. Durch eine derartige Bestimmung der Bildwinkeleigenschaften sind keine Angaben von Seiten des Herstellers nötig.

Zur Durchführung des Verfahrens können durch das mobile Endgerät entsprechende Anweisungen an eine Person, die das Verfahren durchführt, ausgegeben werden. Dies kann beispielsweise über einen Lautsprecher des mobilen Endgeräts oder eine Anzeige des mobilen Endgeräts geschehen. Beispielsweise kann die Person, die das Verfahren durchführt, angewiesen werden, das mobile Endgerät von der ersten Position in die zweite Position zu bewegen. Es ist zu bemerken, dass die Person, die das Verfahren durchführt, identisch mit der Person sein kann, deren Zentrierdaten bestimmt werden, sodass kein Optiker benötigt wird. Durch das Ausgeben der Anweisungen kann auch eine ungeschulte Person das Verfahren durchführen.

Das Verfahren kann weiter ein Messen einer Orientierung des mobilen Endgeräts an der ersten Position, an der zweiten Position und/oder während der Bewegung von der ersten Position in die zweite Position umfassen. Die Orientierung gibt dabei an, wie das mobile Endgerät an der Position, an der es sich jeweils befindet, ausgerichtet ist, und gibt beispielsweise eine Neigung des mobilen Endgeräts an. Die Orientierung kann beispielsweise durch drei Winkel zur Achse eines festen Koordinationssystems angegeben werden. Die Orientierung des mobilen Endgeräts entspricht dann der Orientierung der Kamera des mobilen Endgeräts. Die Kombination aus Orientierung und Position wird manchmal, insbesondere in der Robotik, als Pose bezeichnet, vgl. DIN EN ISO 8373:2012.

Bei manchen Ausführungsbeispielen kann die Bestimmung des mindestens einen Zentrierparameters dann zusätzlich auf Basis der Orientierung z.B. an der ersten Position und/oder zweiten Position vorgenommen werden. Hierdurch können Effekte, die sich durch die Orientierung ergeben, ausgeglichen werden. Beispielsweise können Abstände in dem aufgenommenen Bild je nach Orientierung verschieden erscheinen, und derartige Effekte können kompensiert werden. Dies beruht im Wesentlichen auf einfachen geometrischen Überlegungen, wie die Augenpartie in Abhängigkeit von der Orientierung auf den Bildsensor der Kamera abgebildet wird.

Bei einem alternativen Ausführungsbeispiel kann eine Nachricht ausgegeben werden, wenn die Orientierung des mobilen Endgeräts von einer vorgegebenen Orientierung abweicht. Diese Nachricht kann eine Warnung sein, verbunden mit Anweisungen, das mobile Endgerät wieder zu der vorgegebenen Orientierung zu bewegen. Die vorgegebene Orientierung ist dabei eine Orientierung, auf der die Bestimmung des mindestens einen Zentrierparameters beruht, das heißt Formeln und dergleichen für die Berechnung des mindestens einen Zentrierparameters gehen von dieser vorgegebenen Orientierung aus. Die vorgegebene Orientierung kann beispielsweise eine senkrechte Ausrichtung einer Anzeige des mobilen Endgeräts sein. In diesem Fall müssen verschiedene Orientierungen nicht rechnerisch berücksichtigt werden, was die Berechnung vereinfacht, sondern die Person, die das Verfahren durchführt, wird angehalten, das mobile Endgerät in der vorgegebenen Orientierung zu erhalten.

Die Bewegung kann eine geradlinige Bewegung zu oder von der Augenpartie weg sein, sodass die erste Position und die zweite Position in zwei verschiedenen Abständen von der Augenpartie aufgenommen werden. Der Person, die das Verfahren durchführt, können hierzu wiederum entsprechende Anweisungen gegeben werden. Dies ermöglicht eine relativ einfache Berechnung, die später unter Bezugnahme auf entsprechende Figuren näher erläutert wird.

Die Bewegung kann auch eine Bewegung im Wesentlichen in einer Ebene vor der Augenpartie sein, beispielsweise eine bogenförmige Bewegung. Hier können dann die Zentrierparameter aus dem ersten Bild und dem zweiten Bild ähnlich einer Triangulation ermittelt werden (vgl. den Artikel "Triangulation (Messtechnik)" in der deutschsprachigen Wikipedia, Stand 13. April 2018).

Zu bemerken ist, dass über die Verwendung der Beschleunigung hinaus auch eine weitergehende Ermittlung von Position und Orientierung des mobilen Endgeräts, d.h. der Pose des mobilen Endgeräts, möglich ist, beispielsweise wie in S.-H- Jung und C. Taylor, "Camera trajectory estimation using inertial sensor measurements and structure from motion results", in Proceedings of the IEEE Computer Society, Conference on Computer Vision and Pattern Recognition, vol. 2, 2001, pp. II-732-II-737" und Gabriel Nützi, Stephan Weiss, Davide Scaramuzza, Roland Stiegwart, "Fusion of IMO and Vision for Absolute Scale Estimation in Monocular SLAM, https://doi.org/10.1007/s10846-010-9490-z" beschrieben. Auch können Techniken der Sensorfusion wie in "Sensor Fusion on Android Devices: A Revolution in Motion Processing (InvenSense, https://www.youtube.com/watch?v=C7JQ7Rpwn2k&feature=youtu.be)" Verwendung finden.

Es können dabei auch mehrere Bilder während der Bewegung als nur das erste Bild und das zweite Bild aufgenommen werden. Aus derartigen mehreren Bildern kann dann ein 3D-Modell des Kopfes erstellt werden, ähnlich wie es bei dem eingangs zitierten Gerät Visufit 1000 von Zeiss auf Basis mehrerer Kamerabilder geschieht. Aus diesem 3D-Modell des Kopfes, gegebenenfalls mit Brillenfassung, können dann wie bei diesem stationären Zentriergerät Zentrierparameter ermittelt werden.

Unter einem Modell, insbesondere 3D-Modell, ist eine dreidimensionale Repräsentation von realen Objekten, in diesem Fall der Augenpartie, gegebenenfalls mit Brillenfassung, zu verstehen, die als Datensatz in einem Speichermedium, beispielsweise einem Speicher eines Computers oder einem Datenträger, vorliegen. Eine solche dreidimensionale Repräsentation kann beispielsweise ein 3D-Netz (englisch "3D mesh"), bestehend aus einem Satz von 3D-Punkten, die auch als Vertices bezeichnet werden, und Verbindungen zwischen den Punkten, die auch als Edges bezeichnet werden. Diese Verbindung bilden im einfachsten Fall ein Dreiecksnetz (englisch triangle mesh). Bei einer derartigen Repräsentation als 3D-Netz wird nur die Oberfläche eines Objekts beschrieben, nicht das Volumen. Das Netz muss nicht notwendigerweise geschlossen sein.

Das 3D-Modell kann dabei ein vereinfachtes 3D-Modell sein, welches die Lage der Pupillen und die Lage der Brillenfassung, letztere gemäß dem Kastensystem dargestellt, angibt. In diesem Fall kann die Lage der Pupillen beim Blick geradeaus in die Ferne indirekt über die Lage des mechanische Augendrehpunktes bestimmt werden, wie später anhand von Figuren noch erläutert werden wird. Der mechanische Augendrehpunkt ist dabei nach https://www.spektrum.de/lexikon/optik/augendrehpunkt/264 derjenige Punkt des Auges, der bei Änderungen der Blickrichtung seine Lage am wenigsten verändert, und liegt gemäß dieser Quelle im Mittel 13,5mm hinter dem vorderen Hornhautscheitelpunkt.

Hierzu können Techniken der simultanen Positionsbestimmung und Kartenerstellung, die aus der Robotik bekannt sind, herangezogen werden, siehe beispielsweise den Wikipedia Artikel "simultaneous localization and mapping" der deutschsprachigen Wikipedia, Stand 14. April 2018, mit den dort gegebenen weiteren Nachweisen.

Auch kann allgemein durch eine mehrmalige Aufnahme von Bildern mit herkömmlichen Herangehensweisen der Fehlerrechnung eine Genauigkeit erhöht werden, indem die mehreren Bilder als mehrere Messungen behandelt werden.

Bei den obigen Ausführungsbeispielen werden eine Ebene eines Bildsensors der Kamera und eine Ebene der Brillenfassung im Wesentlichen parallel gehalten. Eine Messung des Vorneigungswinkels (definiert in DIN EN ISO 1366 6: 2012; S.18) ist nicht zielführend möglich. Bei manchen Ausführungsbeispielen kann der Vorneigungswinkel eingegeben werden, und kann in das bestimmte 3D-Modell einfließen. Gleiches gilt für den Hornhaut-Scheitelabstand gemäß DIN EN ISO 1366 6: 2012; S. 27, welcher ebenso separat eingegeben werden kann.

Bei anderen Ausführungsbeispielen kann der Vorneigungswinkel auch gemessen werden, indem das mobile Endgerät beispielsweise in einer Halbkugel oder anderen dreidimensionalen Bewegungen vor dem Gesicht der Augenpartie bewegt wird und so ein 3D-Modell erstellt werden kann, welches den Vorneigungswinkel berücksichtigt. Eine dreidimensionale Bewegung ist dabei eine Bewegung, die Komponenten in drei linear unabhängigen Raumrichtungen umfasst.

Auch wenn die oben beschriebenen Verfahren ohne Messbügel oder anderen Maßstab durchführbar sind, kann bei manchen Ausführungsbeispielen ein derartiger Maßstab bereitgestellt sein, welcher bei der Augenpartie der Person anzubringen ist. Ein Maßstab ist dabei eine Einrichtung, dessen Größe bekannt ist und der daher in dem ersten Bild oder zweiten Bild als Größenreferenz dienen kann. Jedoch kann die Genauigkeit der beschriebenen Verfahren noch erhöht werden, insbesondere bei der Erstellung eines 3D-Modells wie oben beschrieben.

Bei anderen Ausführungsbeispielen kann eine Abmessung von der Person getragenen Brillenfassung durch eine Eingabe auf dem mobilen Endgerät bereitgestellt werden. Eine solche Abmessung kann die Höhe oder Breite der Brillenfassung im Kastensystem sein. Diese kann von einem Hersteller der Brillenfassung angegeben werden oder manuell abgemessen werden. Dies stellt ebenfalls einen Maßstab für das erste Bild und das zweite Bild bereit, indem die Brillenfassung in diesen wie oben erläutert identifiziert wird und die Abmessung in dem Bild mit der eingegebenen Abmessung in Relation gesetzt wird. Auch dies ist jedoch optional, und bei anderen Ausführungsbeispielen wird weder eine Eingabe einer Abmessung noch ein externer Maßstab benötigt.

Gemäß einem weiteren Ausführungsbeispiel wird ein mobiles Endgerät bereitgestellt, umfassend einen Prozessor, eine Kamera und einen Beschleunigungssensor. Der Prozessor ist dabei programmiert, beispielsweise durch ein in einem Speicher des mobilen Endgeräts abgelegtes Computerprogramm, eines der oben beschriebenen Verfahren durchzuführen, das heißt zu bewirken, dass ein erstes Bild einer Augenpartie an einer ersten Position des mobilen Endgeräts aufgenommen wird, ein zweites Bild der Augenpartie an einer zweiten Position des mobilen Endgeräts aufgenommen wird und Zentrierparameter auf Basis des ersten Bilds und des zweiten Bilds bestimmt werden. Dabei wird eine Beschleunigung des mobilen Endgeräts bei der Bewegung von der ersten Position in die zweite Position wiederholt gemessen, und die Bestimmung des Zentrierparameters basiert zudem auf Basis des wiederholten Messens der Beschleunigung.

Schließlich wird auch ein Computerprogramm für ein mobiles Endgerät, das heißt eine App, bereitgestellt, welche, wenn sie auf dem mobilen Endgerät ausgeführt wird, bewirkt, dass eines der oben beschriebenen Verfahren ausgeführt wird. Dieses Computerprogramm kann auf einem nichtflüchtigen Datenträger wie einer Speicherkarte, einer Festplatte, einem optischen Datenträger wie einer DVD oder CD und dergleichen gespeichert sein oder durch ein Datenträgersignal übertragen werden.

Die bestimmten Zentrierparameter können dann zum Einschleifen eines Brillenglases in für sich genommen bekannter Weise verwendet werden. Dementsprechend wird auch ein Verfahren zum Einschleifen eines Brillenglases unter Verwendung des mindestens einen Zentrierparameters, welcher mit einem der obigen Verfahren bestimmt wird, bereitgestellt.

Zur weiteren Erläuterung werden im Folgenden Ausführungsbeispiele der vorliegenden Erfindung unter Bezugnahme auf die beigefügten Zeichnungen näher erläutert. Es zeigen:

Fig. 1 ein Blockdiagramm eines mobilen Endgeräts, wie es bei Ausführungsbeispielen Verwendung findet,

Fig. 2 ein Flussdiagramm eines Verfahrens zur Bestimmung von mindestens einem Zentrierparameter gemäß einem Ausführungsbeispiel,

Fig. 3 ein Diagramm zur Veranschaulichung der Bestimmung von Bildwinkeleigenschaften einer Kamera,

Fig. 4 ein Diagramm zur Veranschaulichung einer möglichen Implementierung der Schritte 21 bis 23 der Fig. 2,

Figuren 5A bis 5C Diagramme zur Veranschaulichung der Bestimmung eines Abstands $\Delta z$ der Fig. 4 auf Basis von Beschleunigungsdaten,

Fig. 6 ein Diagramm zur Veranschaulichung des Bestimmens eines Pupillenabstands bei der Implementierung der Fig. 4,

Fig. 7 ein Diagramm zur Veranschaulichung einer weiteren möglichen Implementierung der Schritte 21 bis 23 des Verfahrens der Fig. 2, und

Fig. 8A und 8B Diagramme zur Veranschaulichung der Bestimmung einer Position des Augendrehpunkts oder der Pupille.

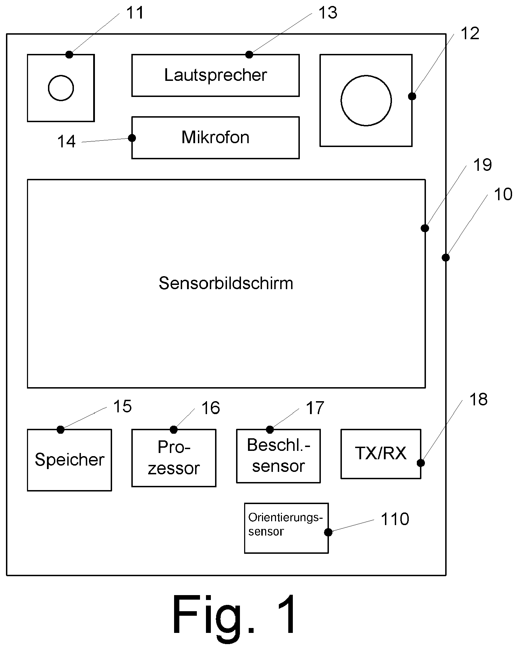

Die im Folgenden beschriebenen Ausführungsbeispiele verwenden ein mobiles Endgerät zur Bestimmung des Pupillenabstands sowie einer Höhe des Kastensystems einer Brillenfassung und gegebenenfalls anderer Zentrierparameter. Die Fig. 1 zeigt ein Blockdiagramm eines mobilen Endgeräts 10, wie es bei derartigen Ausführungsbeispielen verwendet werden kann. Das mobile Endgerät kann dabei ein Smartphone oder ein Tablet-Computer sein. Heutzutage erhältliche Smartphones oder Tablet-Computer weisen typischerweise zumindest die in Fig. 1 dargestellten Komponenten auf, können aber auch weitere Komponenten aufweisen.

Das mobile Endgerät 10 der Fig. 1 weist einen Sensorbildschirm 19 (englisch "Touchscreen") auf, welcher als Eingabegerät sowie zur Ausgabe beispielsweise von Anweisungen an eine Person dient. Gesteuert wird das mobile Endgerät 10 von einem Prozessor 16, der auf einen Speicher 15 zugreifen kann, in dem Computerprogramme abgelegt sein können. Wie bereits eingangs erwähnt, werden derartige Computerprogramme für mobile Endgeräte auch als Apps bezeichnet. Bei dem mobilen Endgerät 10 ist ein Computerprogramm zur Durchführung eines der nachfolgend beschriebenen Verfahren in dem Speicher 15 gespeichert.

Das mobile Endgerät 10 verfügt weiterhin über einen Lautsprecher 13 zur Ausgabe von Tönen und über ein Mikrofon 14. Über den Lautsprecher 13 können Anweisungen an eine Person, die das Verfahren durchführt, ausgegeben werden, und über das Mikrofon 14 können beispielsweise Sprachbefehle entgegengenommen werden.

Das mobile Endgerät 10 weist weiter eine Frontkamera 11 und eine Rückkamera 12 auf. Die Frontkamera 11 ist dabei auf der gleichen Seite wie der Sensorbildschirm 19 angeordnet, sodass mittels der Frontkamera 11 eine Person, insbesondere die Augenpartie einer Person, die den Sensorbildschirm 19 betrachtet, aufgenommen werden kann. Die Rückkamera 12 ist auf der dem Sensorbildschirm 19 gegenüberliegenden Seite des mobilen Endgeräts 10 angeordnet.

Des Weiteren weist das mobile Endgerät 10 einen Beschleunigungssensor 17, mit welchem Beschleunigungen des mobilen Endgeräts 10 gemessen werden können, sowie einen Orientierungssensor 110, mit welchem eine Orientierung des mobilen Endgeräts 10 gemessen werden kann, auf. Ein derartiger Orientierungssensor wird manchmal auch als Neigungssensor bezeichnet. Es ist zu bemerken, dass der Beschleunigungssensor 17 und der Orientierungssensor 110 in der Fig. 1 als separate Komponenten dargestellt sind, um die verschiedenen Funktionen, die im Rahmen der nachfolgend beschriebenen Verfahren verwendet werden, zu veranschaulichen. Die Funktionen können jedoch auch durch eine gemeinsame Sensoreinrichtung bereitgestellt sein.

Schließlich ist eine Kommunikationsschaltung 10 zum Senden (TX, vom englischen "Transmitter") und empfangen (RX, vom englischen "Receiver") von Daten bereitgestellt, beispielsweise über ein Mobilfunknetz und/über ein WLAN-Netz ("Wireless LAN"). Über diese Kommunikationsschaltungen können die bestimmten Zentrierdaten beispielsweise an einen Optiker übermittelt werden, der diese dann zum Einschleifen von Brillengläsern verwendet.

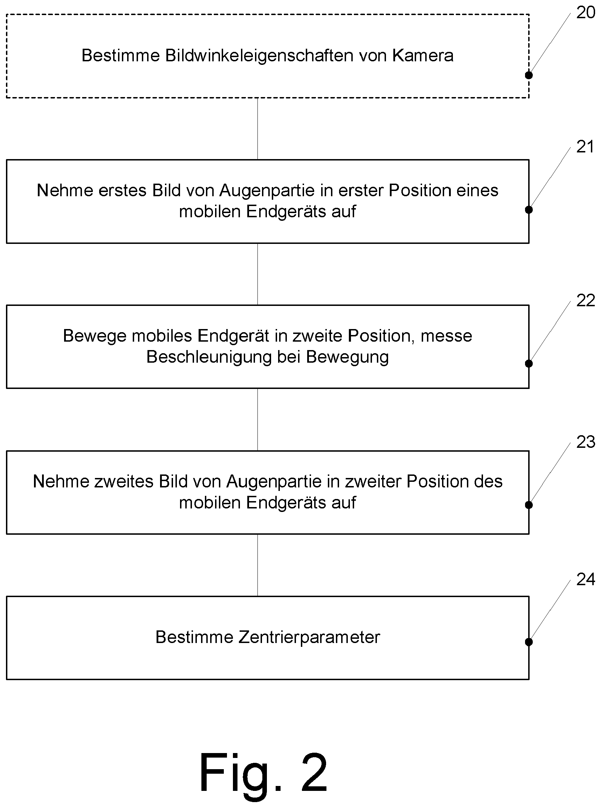

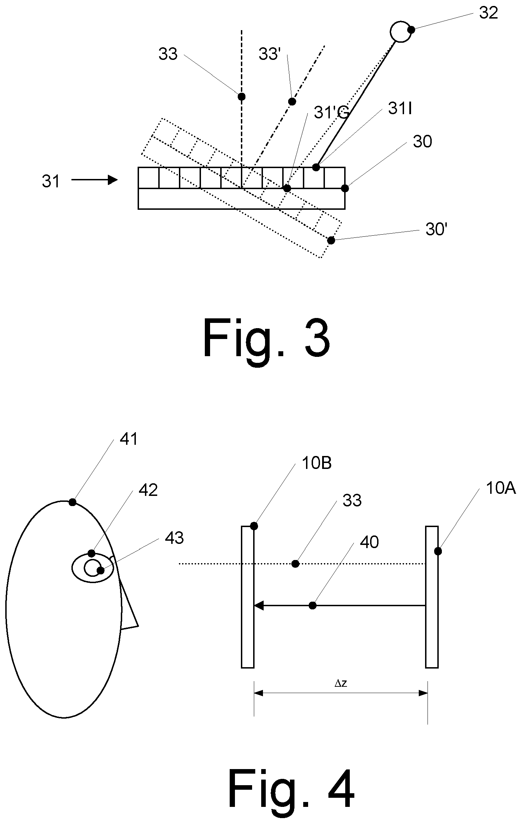

Die Fig. 2 zeigt ein Flussdiagramm zur Veranschaulichung eines Verfahrens gemäß einem Ausführungsbeispiel, welches durch entsprechende Programmierung des Prozessors 16 des mobilen Endgeräts 10 realisiert wird. Zur Veranschaulichung der Schritte des Verfahrens der Fig. 2 wird auf die Figuren 3 bis 7 Bezug genommen.

In Schritt 20 können optional die Bildwinkeleigenschaften einer für die nachfolgenden Schritte verwendeten Kamera, d.h. der Frontkamera 11 oder der Rückkamera 12, des mobilen Endgeräts, bestimmt werden. Diese Bestimmung muss nur einmal durchgeführt werden und wird nicht bei jeder Durchführung der folgenden Schritte durchgeführt. Alternativ zu dem optionalen Schritt 20 können die Bildwinkeleigenschaften auch auf andere Art und Weise erlangt werden. Beispielsweise können die Bildwinkeleigenschaften als Herstellerangaben bereits vorliegen und dann eingegeben werden, oder sie können in manchen Fällen auch herstellerseitig in dem mobilen Endgerät gespeichert sein und dann aus dem Speicher ausgelesen werden.

Eine Implementierung des Schrittes 20 wird anhand der Fig. 3 erläutert. Die Fig. 3 zeigt dabei einen Bildsensor 30 der Kamera 11 oder 12, welcher eine Vielzahl von Bildpunkten 31 aufweist, in einer Querschnittsansicht. Zum Zwecke der Veranschaulichung sind zehn Bildpunkte (üblicherweise als Pixel bezeichnet) 31 dargestellt, welche von links nach rechts mit A bis J (das heißt 31A bis 31J) bezeichnet werden. Auflösungen typischer Kameras von mobilen Endgeräten liegen dabei im Bereich mehrerer Megapixel, sodass die Darstellung der Fig. 3 vereinfacht ist, um die Vorgehensweise besser veranschaulichen zu können.

Zunächst wird ein Bild mit mehreren Objekten in einer ersten Orientierung des Bildsensors 30 aufgenommen. Als Beispiel ist hier ein Objekt 32 dargestellt. Die aufgenommenen Objekte wie das Objekt 32 befinden sich bevorzugt in größerer Entfernung von dem Bildsensor 30, beispielsweise >1m, >5m oder >10m, um Fehler aufgrund von Kamerabewegungen zu minimieren. Das Objekt 32 wird in dem vereinfachten Beispiel der Fig. 3 durch eine (nicht dargestellte) Optik auf den Bildpunkt 31I abgebildet. Mit 33 ist die optische Achse der Kamera bezeichnet.

Dann wird die Kamera um eine Achse gedreht, die senkrecht zur Bildebene der Fig. 3 steht. Der Bildsensor 30 in dieser neuen Position ist mit 30' bezeichnet. In dieser gedrehten Position weist das Objekt nun einen anderen Winkel zu der optischen Achse 33 auf (für die Position 30' mit 33' bezeichnet), und das Objekt 32 wird nunmehr auf den Bildpunkt 31'G abgebildet. Das Objekt 32 kann in beiden aufgenommenen Bildern durch die bereits erwähnten Verfahren zur Bildanalyse erkannt werden. Dies kann für eine Vielzahl derartiger Objekte 32 geschehen. Aus den Verschiebungen der Objekte zwischen den beiden Bildern, d.h. die Änderungen, auf welche Bildpunkte die Objekte abgebildet werden, können dann die Bildwinkeleigenschaften der Kamera bestimmt werden. Der Winkel, um die die Kamera gedreht wird, wird dabei mit dem Orientierungssensor 110 gemessen. Die dargestellte Drehung kann dabei zweimal erfolgen, nämlich um die in der Fig. 3 dargestellte Drehung um eine Achse senkrecht zur Bildebene und um eine Achse, die senkrecht hierzu steht und ebenfalls parallel zu einer Oberfläche des Bildsensors 30 ist. Somit können die Bildwinkeleigenschaften in zwei zueinander senkrechten Richtungen und somit für einen gesamten - üblicherweise - rechteckigen Bildsensor in Schritt 20 ermittelt werden. Die Bestimmung der Bildwinkeleigenschaften kann dabei durch Verzerrungen wie kissenförmige oder tonnenförmige Verzeichnung bei der Bildaufnahme beeinträchtigt werden. Üblicherweise weisen mobile Endgeräte hier jedoch eine intrinsische Kalibrierung auf, die derartige Verzeichnungen zumindest weitgehend rechnerisch beseitigt.

Um zu vermeiden dass ungewollte Translationen der Kamera und somit des Bildsensors 30 während der Drehung der Kamera das Ergebnis verfälschen, kann eine der Drehung der Kamera etwaig überlagerte Translation der Kamera detektiert und rechnerisch von der Drehung separiert werden, um so einen Einfluss der Translation zu kompensieren. Hierzu können Messwerte des Beschleunigungssensors 17 des mobilen Endgeräts und/oder die aufgenommenen Bilder herangezogen werden können. Erleichtert wird diese Kompensation, wenn sich die aufgenommenen Objekte wie das Objekt 32 in unterschiedlichen Distanzen zur Kamera in deren Bildfeld befinden. Die Berechnung der Translation entsprechend einer Positionsänderung kann dabei wie in den oben genannten Referenzen Jung et al. oder Nützi et al. erfolgen.

In Schritt 21 der Fig. 2 wird ein erstes Bild von einer Augenpartie in einer ersten Position des mobilen Endgeräts aufgenommen. In Schritt 22 wird das mobile Endgerät dann in eine zweite Position bewegt, wobei die Beschleunigung bei der Bewegung von der ersten Position zu der zweiten Position gemessen wird. In Schritt 23 wird dann ein zweites Bild von der Augenpartie in einer zweiten Position des mobilen Endgeräts aufgenommen.

Eine mögliche Implementierung dieser Schritte 21-23 ist in der Fig. 4 veranschaulicht. Bei der Implementierung der Fig. 4 wird zunächst in einer ersten Position des mobilen Endgeräts 10, in Fig. 4 mit 10A bezeichnet, ein Bild einer Augenpartie einer Person 41 aufgenommen, von der ein Kopf dargestellt ist. Mit dem Bezugszeichen 43 ist das rechte Auge der Person bezeichnet, die in diesem Fall eine Brillenfassung 42 trägt. Wenn die Person 41 das Verfahren durchführt, erfolgt die Bildaufnahme zweckmäßiger Weise mit der Frontkamera 11 des mobilen Endgeräts, damit die Person gleichzeitig zu der Bewegung den Sensorbildschirm 19 und darauf gegebene Anweisungen und Hinweise betrachten kann. Wird das Verfahren von einer anderen Person, beispielsweise einem Optiker durchgeführt, erfolgt die Bildaufnahme zweckmäßiger Weise mit der Rückkamera 12, sodass dann die andere Person den Sensorbildschirm 19 betrachten kann. Bei der Implementierung der Fig. 4 wird dabei das mobile Endgerät so gehalten, dass die optische Achse 33 auf den Kopf 41 gerichtet ist, insbesondere in etwa auf die Augenpartie. Diese Ausrichtung kann mittels des Orientierungssensors 110 geprüft werden, und beispielsweise bei einer Orientierung des mobilen Endgeräts 10 aus der in Fig. 4 dargestellten Position kann ein Hinweis an die Person, die das Verfahren ausführt, ausgegeben werden, das mobile Endgerät 10 wieder korrekt auszurichten.

Dann wird das mobile Endgerät 10 wie durch einen Pfeil 40 angedeutet parallel zur Richtung der optischen Achse 33 auf den Kopf 41 in eine mit 10B bezeichnete zweite Position bewegt, und hier wird das zweite Bild aufgenommen. Die korrekte Richtung der Bewegung kann wiederum mittels der Sensoren des mobilen Endgeräts 10 überprüft werden und gegebenenfalls ein Hinweis an die Person, die das Verfahren durchführt, ausgegeben werden. Während der Bewegung wird mittels des Beschleunigungssensors 17 die Beschleunigung des mobilen Endgeräts 10 gemessen. Ein Abstand zwischen der ersten Position und der zweiten Position ist in Fig. 4 mit $\Delta z$ bezeichnet. Die Kamera wird dabei in etwa in Höhe der Pupillen gehalten, und die Augen 43 blicken auf die Kamera. In dieser Weise passt die optische Achse der Kamera zu der Blickrichtung der Person.

Bei der Implementierung der Fig. 4 kann zur Erhöhung der Genauigkeit eine Bildaufnahme auch wiederholt während der Bewegung erfolgen.

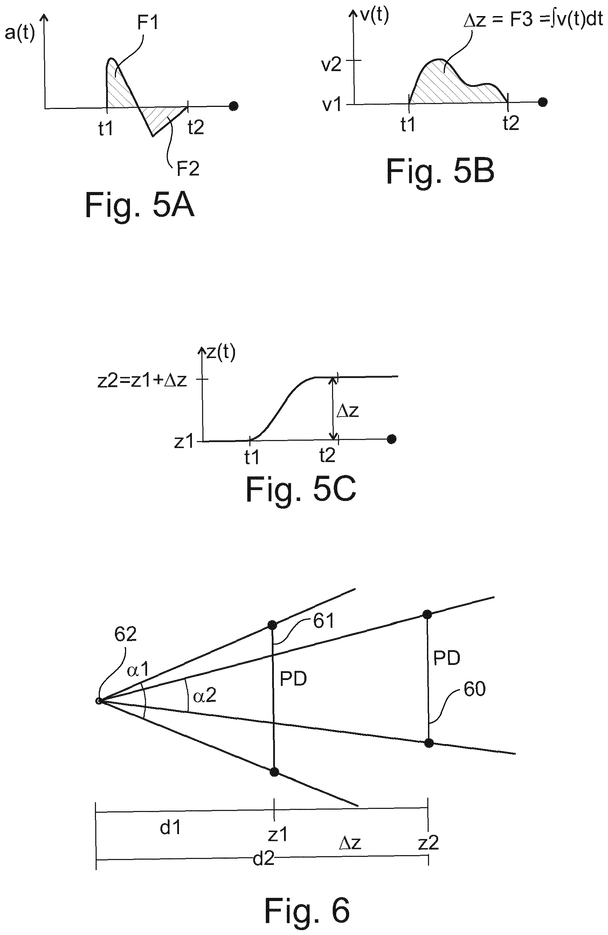

In Schritt 24 werden dann Zentrierparameter aus dem ersten Bild, dem zweiten Bild und der gemessenen Beschleunigung unter Benutzung der Bildwinkeleigenschaften der Kamera bestimmt. Für die Implementierung der Fig. 4 wird dies nun unter Bezugnahme auf die Figuren 5A bis 5C sowie 6 erläutert.

Zur Bestimmung von Zentrierparametern wird zunächst der Abstand $\Delta z$ zwischen der ersten Position und der zweiten Position auf Basis der gemessenen Beschleunigung ermittelt. Dies wird für die Implementierung der Fig. 4 anhand der Figuren 5A bis 5C erläutert.

Die Fig. 5A zeigt ein Beispiel für die Beschleunigung a(t) über der Zeit t für die Bewegung von der ersten Position 10A zu der zweiten Position 10B der Fig. 4. Die Bewegung dauert dabei von einer Zeit t1 zu einer Zeit t2. Zunächst ist die Beschleunigung positiv, wenn das mobile Endgerät 10 von der ersten Position 10A beschleunigt wird, und dann negativ, wenn das mobile Endgerät 10 wieder abgebremst wird, um bei der zweiten Position 10B zur Ruhe zu kommen. Der Bereich, in dem die Beschleunigung positiv ist, ist mit F1, und der Bereich, in dem die Beschleunigung negativ ist, mit F2 bezeichnet.

Durch Integration der Beschleunigung ergibt sich die Geschwindigkeit, wie sie in Fig. 5B gezeigt ist. Zu Beginn der Bewegung (v1) und zum Ende der Bewegung (v2) ist das mobile Endgerät in Ruhe, sodass die Integrationskonstante zur Bestimmung der Geschwindigkeit auf null gesetzt werden kann. Die Fläche F3, das heißt das Integral der Geschwindigkeit über der Zeit, ergibt dann den gewünschten Abstand $\Delta z$, wie dies in Fig. 5C dargestellt ist. Die z-Position in der Position 10A, in Fig. 5C mit z1 bezeichnet, kann dabei als 0 angenommen werden, da für die nachfolgende Bestimmung nur der Wert $\Delta z$ von Interesse ist und die absolute Position nicht benötigt wird.

Die Berechnung von Zentrierparametern wird am Beispiel des Pupillenabstands anhand der Fig. 6 erläutert. Mit 60 ist dabei der Pupillenabstand, wie er in dem ersten Bild an der ersten Position 10A erscheint, bezeichnet, und mit 61 ist der Pupillenabstand in dem zweiten Bild, wie er an der zweiten Position 10B erscheint, bezeichnet. Die jeweilige Position des mobilen Endgeräts ist mit 62 bezeichnet. In Fig. 6 ist also zur Veranschaulichung der geometrischen Verhältnisse die Position des mobilen Endgeräts fest mit 62 bezeichnet, und die verschiedenen Abstände zu dem Kopf 41 sind durch verschiedene Abstände der Pupillendistanz 60 bzw. 61 von diesem Punkt 62 dargestellt.

Der Abstand zwischen dem mobilen Endgerät an der ersten Position 10A zu dem Kopf 41 ist mit D2 bezeichnet, und der Abstand an der zweiten Position 10B zu dem Kopf 41 ist mit D1 bezeichnet. Anhand der oben diskutierten Bildwinkeleigenschaften kann dann aus den Positionen der Pupillen in dem ersten Bild und dem zweiten Bild, das heißt daraus, auf welchen Bildpunkten die Pupillen im ersten Bild und im zweiten Bild erscheinen, ein Winkel α2 für das erste Bild und ein Winkel α1 für das zweite Bild bestimmt werden, unter dem der Pupillenabstand gesehen von der Kamera erscheint, wie dies in Fig. 6 gekennzeichnet ist.

Aus den Werten α1, α2 und Δz, die oben bestimmt wurden, lässt sich der Pupillenabstand PD wie folgt berechnen:

$$\tan(\alpha2/2) = \frac{PD/2}{d2} = \frac{PD/2}{d1 + \Delta z}$$

$$\tan(\alpha1/2) = \frac{PD/2}{d1}$$

$$\frac{1}{\tan(\alpha2/2)} - \frac{d1 + \Delta z}{\frac{PD}{2}} = \frac{1}{\tan(\alpha1/2)} - \frac{d1}{PD/2}$$

$$\left(\frac{1}{\tan(\alpha2/2)} - \frac{1}{\tan(\alpha1/2)}\right)^{-1} * \Delta z * 2 = PD$$

Andere geometrische Größen aus dem ersten Bild und dem zweiten Bild wie die Höhe der Brillenfassung im Kastensystem oder die Breite können in gleicher Weise berechnet werden. In diesem Fall wird dann in den obigen Gleichungen PD durch die entsprechende geometrische Größe ersetzt. Auf diese Weise können Zentrierparameter als Abmessungen in dem ersten und zweiten Bild auf einfache Weise bestimmt werden.

Zudem können nach der Berechnung von PD aus den obigen Gleichungen auch die Werte d1, d2 berechnet werden. Somit ist dann der Abstand von der Kamera des mobilen Endgeräts zu den Pupillen bekannt. Gleiches kann für die Brillenfassung erfolgen. Aus der Differenz zwischen dem Abstand Kamera-Pupille und Kamera-Brillenfassung kann als Differenz der beiden Abstände zudem der Hornhaut-Scheitelabstand als weiterer Zentrierparameter berechnet werden.

Alternativ kann auch ein fester Wert für den Hornhaut-Scheitelabstand angenommen werden, oder dieser kann aus anderen Quellen eingegeben werden.

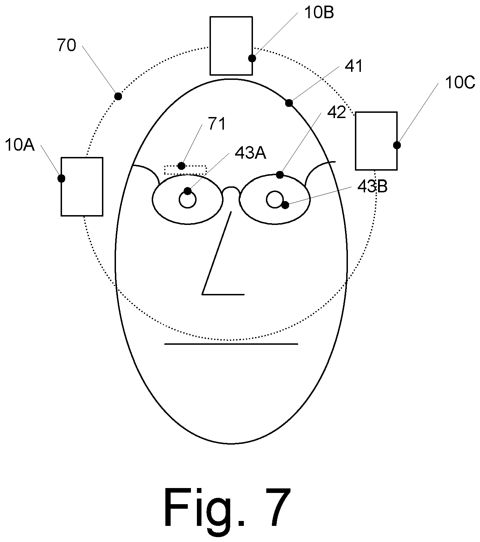

Die Fig. 7 zeigt eine alternative Implementierung für die Schritte 21 bis 23 der Fig. 2. Hier wird das mobile Endgerät in einer Ebene vor der Augenpartie des Kopfes 41 bewegt, und es werden in mindestens zwei Positionen Bilder der Augenpartie aufgenommen. Als Beispiel sind in der Fig. 7 drei Positionen 10A, 10B, 10C entlang einer Kreisbahn 70 gezeigt. Durch Messen der dreidimensionalen Beschleunigung bei der Bewegung zwischen den Positionen kann die relative Lage der Positionen bestimmt werden. Dabei muss keine Kreisbahn wie in Fig. 7 vorliegen, sondern es kann auch eine andere Bahn, beispielsweise eine andere bogenförmige Bahn, verwendet werden.

Die Zentrierdaten, wie der Pupillenabstand können dann in Schritt 24 im Wesentlichen wie bei einer Triangulation bestimmt werden, wie dies bereits eingangs erläutert wurde. Hierzu kann auch die Orientierung des mobilen Endgeräts, das heißt die Orientierung, in jeder der Positionen, in der ein Bild aufgenommen wird, herangezogen werden.

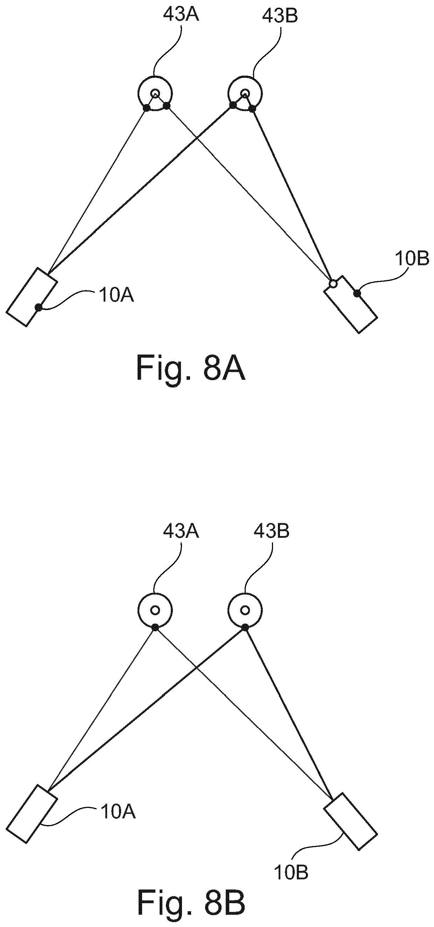

Durch die Aufnahmen der Fig. 7 kann ein vereinfachtes 3D-Modell umfassend die Brillenfassung, repräsentiert durch die Maße im Kastensystem, und die Augen (repräsentiert durch die Pupillen, oder durch die Augendrehpunkte) erstellt werden. Die Person blickt dabei dauerhaft in eine definierte Richtung, z.B. direkt in die Kamera oder einen bekannten, festen Punkt in der Ferne. So können die Positionen der Augendrehpunkte oder der Pupillen bestimmt werden. Ist dies der Fall, können damit die Positionen der Augendrehpunkte und hieraus die Positionen der Pupillen und somit der Pupillenabstand oder direkt die Positionen der Pupillen beider Augen bestimmt werden. Diese beiden Möglichkeiten werden nun unter Bezugnahme auf die Figuren 8A und 8B erläutert.

Die Figur 8A zeigt einen Fall, in dem die Person auf das mobile Endgerät 10 blickt, das in zwei Positionen 10A, 10B dargestellt ist. Die Pupillen der Augen 43A, 43B sind als schwarze Punkte dargestellt und folgen in diesem Fall der Position des mobilen Endgeräts 10, d.h. sie sind auf das mobile Endgerät 10 in der jeweiligen Position 10A oder 10B gerichtet. Wenn nun eine Triangulation wie oben erwähnt anhand der Lage der Pupillen in den jeweils in den Positionen 10A, 10B aufgenommenen Bildern durchgeführt wird, ergeben sich als Ergebnis die Positionen der Augendrehpunkte, wie aus der Fig. 8A ersichtlich, entsprechend der Schnittpunkte der Verbindungslinien von den Positionen 10A, 10B zu den jeweiligen Positionen der Pupillen. Hieraus kann dann die Position der Pupillen beim Blick geradeaus in die Ferne abgeschätzt werden, indem ein Wert von ca. 13,5mm als mittlerer Abstand des Augendrehpunkts vom vorderen Hornhautscheitel von der Position der Augendrehpunkte in frontaler Richtung der Person hinzuaddiert wird. Aus den so abgeschätzten Positionen der Pupillen kann wiederum der Pupillenabstand bestimmt werden.

In Fig. 8B ist der Fall dargestellt, in dem die Person geradeaus auf ein Ziel in der Ferne blickt, während das mobile Endgerät in den Positionen 10A, 10B Bilder aufnimmt. In diesem Fall folgen die Pupillen dem mobilen Endgerät 10 nicht, und es wird durch Triangulation direkt die Position der Pupillen bestimmt.

Näheres zu derartigen Positionsberechnungen auf Basis von Bildaufnahmen ist auch der US 2013/083976 A, der US 2013/076884 A oder der WO 15/101737 A1 zu entnehmen.

Die Bildaufnahme kann sowohl bei der Fig. 3 als auch bei der Fig. 7 auch wiederholt erfolgen, und entsprechend mehrerer Bilder kann dann die Genauigkeit der Bestimmung der Zentrierparameter entsprechend einer herkömmlichen Fehlerrechnung erhöht werden. Bei der Herangehensweise der Fig. 7 kann insbesondere ein 3D-Modell erstellt werden.

Wie bereits eingangs erwähnt ist bei den Verfahren zwar kein Messbügel oder anderer Maßstab notwendig. Optional kann ein solcher Maßstab, welcher in der Fig. 7 mit dem Bezugszeichen 71 bezeichnet ist, zusätzlich als Größenmaßstab bereitgestellt werden, um die Genauigkeit der Bestimmung zu erhöhen. Insbesondere kann dies die Erstellung des 3-D Modells im Falle der Fig. 7 unterstützen.

Manche Ausführungsbeispiele werden durch die nachfolgenden Klauseln definiert:

Klausel 1. Computerimplementiertes Verfahren für ein mobiles Endgerät zum Bestimmen mindestens eines Zentrierparameters, umfassend:

Aufnehmen eines ersten Bildes einer Augenpartie einer Person an einer ersten Position des mobilen Endgeräts mit einer Kamera des mobilen Endgeräts,
Aufnehmen eines zweiten Bildes der Augenpartie an einer zweiten Position des mobilen Endgerätes mit der Kamera, und
Bestimmen des mindestens einen Zentrierparameters auf Basis des ersten Bildes und des zweiten Bildes, gekennzeichnet durch
wiederholtes Messen einer Beschleunigung des mobilen Endgerätes bei einer Bewegung des mobilen Endgerätes von der ersten Position zu der zweiten Position,
wobei das Bestimmen des mindestens einen Zentrierparameters zudem auf Basis des wiederholten Messens der Beschleunigung erfolgt.

Klausel 2. Verfahren nach Klausel 1, dadurch gekennzeichnet, dass das Bestimmen des mindestens einen Zentrierparameters zudem auf Basis von Bildwinkeleigenschaften der Kamera erfolgt.

Klausel 3. Verfahren nach Klausel 2, dadurch gekennzeichnet, dass das Verfahren zudem ein Bestimmen der Bildwinkeleigenschaften der Kamera umfasst.

Klausel 4. Verfahren nach einer der Klauseln 1 bis 3, gekennzeichnet durch ein Ausgeben von Anweisungen durch das mobile Endgerät an eine Person zum Bewegen des mobilen Endgeräts von der ersten Position zu der zweiten Position.

Klausel 5. Verfahren nach einer der Klauseln 1 bis 4, gekennzeichnet durch:

Messen einer Orientierung des mobilen Endgeräts,
wobei das Bestimmen des Zentrierparameters zudem auf der Orientierung des mobilen Endgeräts basiert.

Klausel 6. Verfahren nach einer der Klauseln 1 bis 4, gekennzeichnet durch:

Messen einer Orientierung des mobilen Endgeräts, und
Ausgeben eines Hinweises, wenn die Orientierung sich von einer vorgegebenen Orientierung unterscheidet.

Klausel 7. Verfahren nach einer der Klauseln 1 bis 6, dadurch gekennzeichnet, dass die Bewegung eine geradlinige Bewegung auf die Augenpartie zu oder von der Augenpartie weg umfasst.

Klausel 8. Verfahren nach einer der Klauseln 1 bis 7, dadurch gekennzeichnet, dass die Bewegung eine Bewegung in einer Ebene vor der Augenpartie umfasst.

Klausel 9. Verfahren nach einer der Klauseln 1 bis 8, dadurch gekennzeichnet, dass die Bewegung eine dreidimensionale Bewegung vor der Augenpartie umfasst.

Klausel 10. Verfahren nach einer der Klauseln 1 bis 9, dadurch gekennzeichnet, dass der mindestens eine Zentrierparameter mindestens einen Parameter aus der Gruppe umfassend einen Pupillenabstand, ein Maß eines Kastensystems, einen Hornhaut-Scheitelabstand und einen Vorneigungswinkel umfasst.

Klausel 11. Verfahren nach einer der Klauseln 1 bis 10, dadurch gekennzeichnet, dass das Verfahren ein Aufnehmen einer Vielzahl von Bildern bei der Bewegung umfassend das erste Bild und das zweite Bild umfasst, wobei das Bestimmen des Zentrierparameters auf Basis der Vielzahl von Bildern erfolgt.

Klausel 12. Verfahren nach einer der Klauseln 1 bis 11, dadurch gekennzeichnet, dass das Verfahren ein Erstellen eines 3D-Modells basierend auf dem ersten Bild und dem zweiten Bild umfasst.

Klausel 13. Verfahren nach einer der Klauseln 1 bis 12, dadurch gekennzeichnet, dass das Verfahren keinen an der Person anzubringenden Maßstab verwendet.

Klausel 14. Verfahren nach einer der Klauseln 1 bis 13, dadurch gekennzeichnet, dass zumindest ein Aufnehmen aus der Gruppe umfassend das Aufnehmen des ersten Bildes und das Aufnehmen des zweiten Bildes ein Aufnehmen eines Maßstabs umfasst, wobei das Bestimmen des mindestens einen Zentrierparameters zudem auf Basis des Maßstabs erfolgt.

Klausel 15. Verfahren nach einer der Klauseln 1 bis 14, dadurch gekennzeichnet, dass das Verfahren weiter ein Empfangen einer Abmessung einer von der Person getragenen Brillenfassung umfasst, wobei ein Verhältnis der Abmessung zu einer entsprechenden Abmessung in zumindest einem Bild aus der Gruppe umfassend das erste Bild und das zweite Bild als Größenmaßstab dient.

Klausel 16. Verfahren zum Einschleifen eines Brillenglases, umfassend:

Bestimmen von mindestens einem Zentrierparameter mit dem Verfahren nach einer der Klauseln 1 bis 15, und Einschleifen des Brillenglases basierend auf dem mindestens einen Zentrierparameter.

Klausel 17. Computerprogramm für ein mobiles Endgerät mit einem Programmcode, der, wenn er auf einem Prozessor des mobilen Endgeräts ausgeführt wird, bewirkt, dass das mobile Endgerät das Verfahren nach einer der Klauseln 1 bis 15 durchführt.

Klausel 18. Mobiles Endgerät zum Bestimmen mindestens eines Zentrierparameters, umfassend:

einen Prozessor, eine Kamera und einen Beschleunigungssensor, wobei der Prozessor konfiguriert ist zum:

Aufnehmen eines ersten Bildes einer Augenpartie einer Person an einer ersten Position des mobilen Endgeräts mit einer Kamera des mobilen Endgeräts, Aufnehmen eines zweiten Bildes der Augenpartie an einer zweiten Position des mobilen Endgerätes mit der Kamera, und Bestimmen des mindestens einen Zentrierparameters auf Basis des ersten Bildes und des zweiten Bildes,

dadurch gekennzeichnet, dass der Prozessor weiter konfiguriert ist zum:

wiederholten Messen einer Beschleunigung des mobilen Endgeräts bei einer Bewegung des mobilen Endgeräts von der ersten Position zu der zweiten Position, wobei das Bestimmen des mindestens einen Zentrierparameters zudem auf Basis des wiederholten Messens der Beschleunigung erfolgt.

Klausel 19. Mobiles Endgerät nach Klausel 18, dadurch gekennzeichnet, dass das Bestimmen des mindestens einen Zentrierparameters zudem auf Basis von Bildwinkeleigenschaften der Kamera erfolgt.

Klausel 20. Mobiles Endgerät nach Klausel 19, dadurch gekennzeichnet, dass der Prozessor weiter konfiguriert ist zum Bestimmen der Bildwinkeleigenschaften der Kamera.

Klausel 21. Mobiles Endgerät nach einer der Klauseln 18 bis 20, dadurch gekennzeichnet, dass der Prozessor weiter konfiguriert ist zum Ausgeben von Anweisungen durch das mobile Endgerät an eine Person zum Bewegen des mobilen Endgeräts von der ersten Position zu der zweiten Position.

Klausel 22. Mobiles Endgerät nach einer der Klauseln 18 bis 21, dadurch gekennzeichnet, dass der Prozessor weiter konfiguriert ist zum Messen einer Orientierung des mobilen Endgeräts,
wobei das Bestimmen des Zentrierparameters zudem auf der Orientierung des mobilen Endgeräts basiert.

Klausel 23. Mobiles Endgerät nach einer der Klauseln 18 bis 22, dadurch gekennzeichnet, dass der Prozessor weiter konfiguriert ist zum Messen einer Orientierung des mobilen Endgeräts, und
Ausgeben eines Hinweises, wenn die Orientierung sich von einer vorgegebenen Orientierung unterscheidet.

Klausel 24. Mobiles Endgerät nach einer der Klauseln 18 bis 23, dadurch gekennzeichnet, dass der mindestens eine Zentrierparameter einen Pupillenabstand, ein Maß eines Kastensystems, einen Hornhaut-Scheitelabstand und/oder einen Vorneigungswinkel umfasst.

Klausel 25. Mobiles Endgerät nach einer der Klauseln 18 bis 24, dadurch gekennzeichnet, dass der Prozessor weiter konfiguriert ist zum Aufnehmen einer Vielzahl von Bildern bei der Bewegung umfassend das erste Bild und das zweite Bild, wobei das Bestimmen des Zentrierparameters auf Basis der Vielzahl von Bildern erfolgt.

Klausel 26. Mobiles Endgerät nach einer der Klauseln 18 bis 25, dadurch gekennzeichnet, dass der Prozessor weiter konfiguriert ist zum Erstellen eines 3D-Modells basierend auf dem ersten Bild und dem zweiten Bild.

Klausel 27. Computerlesbarer nichtflüchtiger Datenträger, der Instruktionen umfasst, die, wenn sie auf einem mobilen Endgerät, welches eine Kamera und einen Beschleunigungssensor umfasst, ausgeführt werden, bewirken, dass das mobile Endgerät folgende Schritte ausführt: Aufnehmen eines ersten Bildes einer Augenpartie einer Person an einer ersten Position des mobilen Endgeräts mit einer Kamera des mobilen Endgeräts,
Aufnehmen eines zweiten Bildes der Augenpartie an einer zweiten Position des mobilen Endgerätes mit der Kamera, und
Bestimmen des mindestens einen Zentrierparameters auf Basis des ersten Bildes und des zweiten Bildes, gekennzeichnet durch
wiederholtes Messen einer Beschleunigung des mobilen Endgerätes bei einer Bewegung des mobilen Endgerätes von der ersten Position zu der zweiten Position,
wobei das Bestimmen des mindestens einen Zentrierparameters zudem auf Basis des wiederholten Messens der Beschleunigung erfolgt.

Klausel 28. Datenträgersignal, das ein Computerprogramm überträgt, das, wenn es auf einem mobilen Endgerät, welches eine Kamera und einen Beschleunigungssensor umfasst, ausgeführt wird, bewirkt, dass das mobile Endgerät folgende Schritte ausführt:

Aufnehmen eines ersten Bildes einer Augenpartie einer Person an einer ersten Position des mobilen Endgeräts mit einer Kamera des mobilen Endgeräts,
Aufnehmen eines zweiten Bildes der Augenpartie an einer zweiten Position des mobilen Endgerätes mit der Kamera, und
Bestimmen des mindestens einen Zentrierparameters auf Basis des ersten Bildes und des zweiten Bildes, gekennzeichnet durch
wiederholtes Messen einer Beschleunigung des mobilen Endgerätes bei einer Bewegung des mobilen Endgerätes von der ersten Position zu der zweiten Position,
wobei das Bestimmen des mindestens einen Zentrierparameters zudem auf Basis des wiederholten Messens der Beschleunigung erfolgt.

Klausel 29. Mobiles Endgerät umfassend einen Prozessor, eine Kamera , einen Beschleunigungssensor und einen Speicher mit einem darin gespeicherten Computerprogramm, wobei der Prozessor eingerichtet ist, basierend auf dem Computerprogramm das mobile Endgerät zur Durchführung folgender Schritte zu steuern: Aufnehmen eines ersten Bildes einer Augenpartie einer Person an einer ersten Position des mobilen Endgeräts mit einer Kamera des mobilen Endgeräts,
Aufnehmen eines zweiten Bildes der Augenpartie an einer zweiten Position des mobilen Endgerätes mit der Kamera, und

Bestimmen des mindestens einen Zentrierparameters auf Basis des ersten Bildes und des zweiten Bildes, gekennzeichnet durch

wiederholtes Messen einer Beschleunigung des mobilen Endgerätes bei einer Bewegung des mobilen Endgerätes von der ersten Position zu der zweiten Position,

wobei das Bestimmen des mindestens einen Zentrierparameters zudem auf Basis des wiederholten Messens der Beschleunigung erfolgt.

## Patentansprüche

1. Computerimplementiertes Verfahren für ein mobiles Endgerät zum Bestimmen mindestens eines Zentrierparameters, umfassend:

   Aufnehmen eines ersten Bildes einer Augenpartie einer Person an einer ersten Position (10A) des mobilen Endgeräts (10) mit einer Kamera (11, 12) des mobilen Endgeräts (10),
   Aufnehmen eines zweiten Bildes der Augenpartie an einer zweiten Position (10B) des mobilen Endgerätes (10) mit der Kamera (11, 12), und
   Bestimmen des mindestens einen Zentrierparameters auf Basis des ersten Bildes und des zweiten Bildes, **gekennzeichnet durch**
   wiederholtes Messen einer Beschleunigung des mobilen Endgerätes (10) bei einer Bewegung des mobilen Endgerätes (10) von der ersten Position zu der zweiten Position,
   wobei das Bestimmen des mindestens einen Zentrierparameters zudem auf Basis des wiederholten Messens der Beschleunigung erfolgt.

2. Verfahren nach Anspruch 1, **dadurch gekennzeichnet, dass** das Bestimmen des mindestens einen Zentrierparameters zudem auf Basis von Bildwinkeleigenschaften der Kamera (11, 12) erfolgt.

3. Verfahren nach Anspruch 2, **dadurch gekennzeichnet, dass** das Verfahren zudem ein Bestimmen der Bildwinkeleigenschaften der Kamera umfasst.

4. Verfahren nach einem der Ansprüche 1 bis 3, **gekennzeichnet durch**:

   Messen einer Orientierung des mobilen Endgeräts (10),
   wobei das Bestimmen des Zentrierparameters zudem auf der Orientierung des mobilen Endgeräts (10) basiert.

5. Verfahren nach einem der Ansprüche 1 bis 3, **gekennzeichnet durch**:

   Messen einer Orientierung des mobilen Endgeräts, und
   Ausgeben eines Hinweises, wenn die Orientierung sich von einer vorgegebenen Orientierung unterscheidet.

6. Verfahren nach einem der Ansprüche 1 bis 5, **dadurch gekennzeichnet, dass** die Bewegung eine geradlinige Bewegung auf die Augenpartie zu oder von der Augenpartie weg umfasst.

7. Verfahren nach einem der Ansprüche 1 bis 6, **dadurch gekennzeichnet, dass** die Bewegung eine Bewegung ausgewählt aus der Gruppe umfassend eine Bewegung in einer Ebene und eine dreidimensionale Bewegung vor der Augenpartie umfasst.

8. Verfahren nach einem der Ansprüche 1 bis 7, **dadurch gekennzeichnet, dass** der mindestens eine Zentrierparameter mindestens einen Parameter aus der Gruppe umfassend einen Pupillenabstand, ein Maß eines Kastensystems,
   einen Hornhaut-Scheitelabstand und einen Vorneigungswinkel umfasst.

9. Verfahren nach einem der Ansprüche 1 bis 8, **dadurch gekennzeichnet, dass** das Verfahren ein Aufnehmen einer Vielzahl von Bildern bei der Bewegung umfassend das erste Bild und das zweite Bild umfasst, wobei das Bestimmen des Zentrierparameters auf Basis der Vielzahl von Bildern erfolgt.

10. Verfahren nach einem der Ansprüche 1 bis 9, **dadurch gekennzeichnet, dass** das Verfahren ein Erstellen eines 3D-Modells basierend auf dem ersten Bild und dem zweiten Bild umfasst.

**11.** Verfahren nach einem der Ansprüche 1 bis 10, **dadurch gekennzeichnet, dass** zumindest ein Aufnehmen aus der Gruppe umfassend das Aufnehmen des ersten Bildes und das Aufnehmen des zweiten Bildes ein Aufnehmen eines Maßstabs umfasst, wobei das Bestimmen des mindestens einen Zentrierparameters zudem auf Basis des Maßstabs erfolgt.

**12.** Verfahren nach einem der Ansprüche 1 bis 11, **dadurch gekennzeichnet, dass** das Verfahren weiter ein Empfangen einer Abmessung einer von der Person getragenen Brillenfassung umfasst, wobei ein Verhältnis der Abmessung zu einer entsprechenden Abmessung in zumindest einem Bild aus der Gruppe umfassend das erste Bild und das zweite Bild als Größenmaßstab dient.

**13.** Verfahren zum Einschleifen eines Brillenglases, umfassend:

Bestimmen von mindestens einem Zentrierparameter mit dem Verfahren nach einem der Ansprüche 1 bis 12, und Einschleifen des Brillenglases basierend auf dem mindestens einen Zentrierparameter.

**14.** Computerprogramm für ein mobiles Endgerät mit einem Programmcode, der, wenn er auf einem Prozessor des mobilen Endgeräts ausgeführt wird, bewirkt, dass das mobile Endgerät das Verfahren nach einem der Ansprüche 1 bis 12 durchführt.

**15.** Mobiles Endgerät zum Bestimmen mindestens eines Zentrierparameters, umfassend:

einen Prozessor, eine Kamera und einen Beschleunigungssensor, wobei der Prozessor konfiguriert ist zum:

Aufnehmen eines ersten Bildes einer Augenpartie einer Person an einer ersten Position (10A) des mobilen Endgeräts (10) mit einer Kamera (11, 12) des mobilen Endgeräts (10),
Aufnehmen eines zweiten Bildes der Augenpartie an einer zweiten Position (10B) des mobilen Endgerätes (10) mit der Kamera (11, 12), und
Bestimmen des mindestens einen Zentrierparameters auf Basis des ersten Bildes und des zweiten Bildes,

**dadurch gekennzeichnet, dass** der Prozessor weiter konfiguriert ist zum:

wiederholten Messen einer Beschleunigung des mobilen Endgeräts (10) bei einer Bewegung des mobilen Endgeräts (10) von der ersten Position zu der zweiten Position,
wobei das Bestimmen des mindestens einen Zentrierparameters zudem auf Basis des wiederholten Messens der Beschleunigung erfolgt.

Fig. 1

Bestimme Bildwinkeleigenschaften von Kamera — 20

Nehme erstes Bild von Augenpartie in erster Position eines mobilen Endgeräts auf — 21

Bewege mobiles Endgerät in zweite Position, messe Beschleunigung bei Bewegung — 22

Nehme zweites Bild von Augenpartie in zweiter Position des mobilen Endgeräts auf — 23

Bestimme Zentrierparameter — 24

# Fig. 2

33    33'    31'G    31I    30
31 →    32    30'

# Fig. 3

41    42    43    10B    33    40    10A

Δz

# Fig. 4

Fig. 5A

Fig. 5B

$\Delta z = F3 = \int v(t) dt$

Fig. 5C

$z2 = z1 + \Delta z$

Fig. 6

Fig. 7

43A          43B

10B

10A

Fig. 8A

43A          43B

10A

10B

Fig. 8B

**Europäisches Patentamt**
**European Patent Office**
**Office européen des brevets**

# EUROPÄISCHER RECHERCHENBERICHT

Nummer der Anmeldung

EP 20 17 6093

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2018/140186 A1 (LIMON OFER [IL]) 24. Mai 2018 (2018-05-24) * Zusammenfassung; Abbildungen 1-11 * * Absätze [0039], [0048], [0049], [0073], [0079], [0082], [0083], [0088], [0089], [0109] - [0114], [0135], [0137], [0143] - [0168] * * Absätze [0177] - [0183], [0197], [0211] - [0214], [0237] - [0247] * ----- | 1-11,14, 15 | INV. G02C13/00 A61B3/11 |
| X | DE 10 2011 009646 A1 (OLLENDORF HANS-JOACHIM [DE]) 2. August 2012 (2012-08-02) * Zusammenfassung; Ansprüche 1,17; Abbildungen 1-17 * * Absätze [0001], [0006], [0027], [0028], [0048], [0054], [0057] - [0076] * ----- | 1,4,5, 7-15 | |
| A | US 2019/196221 A1 (EL-HAJAL BASSEM [CA] ET AL) 27. Juni 2019 (2019-06-27) * Zusammenfassung; Abbildungen 1-7 * * Absätze [0005] - [0008], [0013], [0014], [0025] - [0032], [0035], [0039] - [0042] * ----- | 1,10,14, 15 | RECHERCHIERTE SACHGEBIETE (IPC) A61B G02C |

Der vorliegende Recherchenbericht wurde für alle Patentansprüche erstellt

| Recherchenort | Abschlußdatum der Recherche | Prüfer |
|---|---|---|
| München | 13. Oktober 2020 | Köhn, Andreas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.................................................................................

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 20 17 6093

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-10-2020

| Im Recherchenbericht angeführtes Patentdokument | Datum der Veröffentlichung | Mitglied(er) der Patentfamilie | Datum der Veröffentlichung |
|---|---|---|---|
| US 2018140186 A1 | 24-05-2018 | AU 2016261275 A1 | 30-11-2017 |
| | | BR 112017024157 A2 | 17-07-2018 |
| | | CA 2985315 A1 | 17-11-2016 |
| | | CN 107847128 A | 27-03-2018 |
| | | EP 3294112 A1 | 21-03-2018 |
| | | JP 2018518233 A | 12-07-2018 |
| | | KR 20180004271 A | 10-01-2018 |
| | | RU 2017139578 A | 11-06-2019 |
| | | SG 10201913884S A | 30-03-2020 |
| | | US 2018140186 A1 | 24-05-2018 |
| | | WO 2016181308 A1 | 17-11-2016 |
| DE 102011009646 A1 | 02-08-2012 | DE 102011009646 A1 | 02-08-2012 |
| | | WO 2012100771 A2 | 02-08-2012 |
| US 2019196221 A1 | 27-06-2019 | CA 3085733 A1 | 27-06-2019 |
| | | US 2019196221 A1 | 27-06-2019 |
| | | WO 2019125700 A1 | 27-06-2019 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9928421 B1
- US 9971172 B1
- US 7950800 B1
- US 8360580 B1
- US 9535270 B2

- US 99771172 B1
- EP 3355214 A1
- US 2013083976 A
- US 2013076884 A
- WO 15101737 A1

**In der Beschreibung aufgeführte Nicht-Patentliteratur**

- **S. KIM et al.** A Fast Center of Pupil Detection Algorithm for VOG-Based Eye Movement Tracking. *Conf Proc IEEE Eng Med Biol Soc.,* 2005, vol. 3, 3188-91
- **MANSOUR ASADIFARD ; JAMSHID SHANBEZA-DEH.** Automatic Adaptive Center of Pupil Detection Using Face Detection and CDF Analysis. *Proceedings of the International MultiConference of Engineers and Computer Scientists 2010,* 17. Marz 2010, vol. I
- **S.-H- JUNG ; C. TAYLOR.** Camera trajectory estimation using inertial sensor measurements and structure from motion results. *Proceedings of the IEEE Computer Society, Conference on Computer Vision and Pattern Recognition,* 2001, vol. 2, II-732-II-737

- **GABRIEL NÜTZI ; STEPHAN WEISS ; DAVIDE SCARAMUZZA ; ROLAND STIEGWART.** *Fusion of IMO and Vision for Absolute Scale Estimation in Monocular SLAM,* https://doi.org/10.1007/s10846-010-9490-z
- *Sensor Fusion on Android Devices: A Revolution in Motion Processing,* https://www.youtube.com/watch?v=C7JQ7Rpwn2k&feature=youtu.be
- simultaneous localization and mapping. *deutschsprachigen Wikipedia,* April 2018